# EUROPEAN PATENT SPECIFICATION

(11) **EP 2 451 469 B1**
(45) Date of publication and mention of the grant of the patent: **20.08.2014**
(21) Application number: 10740272.9
(22) Date of filing: 05.07.2010
(51) Int. Cl.: A61K 38/09, A61P 5/04, A61P 5/24

(54) **Composition for the treatment of benign prostate hyperplasia**
Zusammensetzung zur Behandlung von gutartiger Prostatahyperplasie
Composition destinée à traiter une hyperplasie prostatique bénigne

(30) Priority: 06.07.2009 EP 09251738; 21.08.2009 US 235816 P
(43) Date of publication of application: 16.05.2012
(73) Proprietor: Ferring B.V., 2132 JX Hoofddorp (NL)
(72) Inventor: PETRI, Axel, Niclas, DK-2400 Copenhagen NV (DK); ERICHSEN Lars, DK-3520 Farum (DK)
(74) Representative: Bates, Philip Ian
(86) International application number: PCT/IB2010/001785
(87) International publication number: WO 2011/004260

(56) References cited:
- EP-A1- 1 674 082
- EP-A1- 1 967 202
- WO-A1-98/46634
- WO-A1-03/006049
- WO-A2-2006/106311
- SORBERA L A ET AL: "Degarelix acetate. GnRH antagonist, prostate cancer therapy", DRUGS OF THE FUTURE, PROUS SCIENCE, ES, vol. 31, no. 9, 1 September 2006 (2006-09-01), pages 755-766, XP002548773, ISSN: 0377-8282, DOI: DOI:10.1358/DOF.2006.031.09.1024366
- "Explorative Study of Degarelix for Treatment of Benign Prostatic Hyperplasia", INTERNET CITATION, 25 June 2009 (2009-06-25), pages 1-4, XP002548774, Retrieved from the Internet: URL:http://www.clinicaltrials.gov/ct2/show /NCT00527488?term=degarelix+and +bph&rank=1 [retrieved on 2009-10-02]
- ANONYMOUS: "Annex I Summary of product characteristics [Firmagon]", INTERNET CITATION, 17 February 2009 (2009-02-17), pages 1-15, XP002548775, Retrieved from the Internet: URL:http://www.emea.europa.eu/humandocs/PD Fs/EPAR/firmagon/emea-combined- h986en.pdf [retrieved on 2009-10-02]
- ENGEL J B ET AL: "Drug Insight: clinical use of agonists and antagonists of luteinizing-hormone-releasing hormone", NATURE CLINICAL PRACTICE ENDOCRINOLOGY & METABOLISM, NATURE PUBLISHING GROUP, LONDON, GB, vol. 3, no. 2, 1 February 2007 (2007-02-01), pages 157-167, XP008112985, ISSN: 1745-8366, DOI: DOI:10.1038/NCPANDMET0399

### SEQUENCE LISTING

<110> FERRING INTERNATIONAL CENTER S.A.
<120> COMPOSITION FOR THE TREATMENT OF BENIGN PROSTATE HYPERPLASIA
<130> P/63955.WO01
<150> EP 09251738.2
   <151> 2009-07-06
<150> US 61/235816
   <151> 2009-08-21
<160> 1
<170> PatentIn version 3.3
<210> 1
   <211> 10
   <212> PRT
   <213> Homo sapiens
<220>
   <221> PYRROLIDONE CARBOXYLIC ACID
   <222> (1)..(1)
   <223> N-terminal glutamate which has formed an internal cyclic lactam (Glu modified to pyroGlu)
<400> 1
   Glu His Trp Ser Tyr Gly Leu Arg Pro Gly
   1               5                   10

## Description

The present invention relates to compositions for the treatment of benign prostatic hyperplasia (BPH), and methods of treatment of BPH using these compositions.

### Background

Benign prostatic hyperplasia (BPH), sometimes known as benign prostatic hypertrophy or benign prostatic obstruction, is a condition where an abnormal proliferation of prostate cells causes a benign enlargement of the organ which may eventually lead to urinary obstruction and lower urinary tract symptoms. According to the United States National Institutes of Health, BPH affects more than 50% of men over age 60 and as many as 90% of men over the age of 70.

BPH may be treated by surgery to remove prostatic tissue. This reduces the physical bulk of the prostate, thereby reducing obstruction and urinary symptoms. Transurethral resection of the prostate (TURP) is the gold standard surgical treatment for urinary symptoms due to BPH. The procedure is effective and tolerated reasonably well, although associated with postoperative morbidity like urinary incontinence and retrograde ejaculation. As a consequence, although TURP is effective, it is considered as a last resort, and medicinal therapy has become the first line treatment of symptomatic BPH.

The primary treatment goals for men with BPH are to alleviate lower urinary tract symptoms and prevent the progression of the disease, in particular the risk of acute urinary retention and need for surgery. The risk of progression is directly related to the prostate volume. Therefore, drugs that reduce prostate volume have been shown to exhibit the greatest effect in preventing the disease progression. Two classes of drugs, α-blockers and 5-α-reductase inhibitors, are presently approved by authorities for treatment of BPH. Only the 5-α-reductase inhibitors, either alone or in combination with α-blockers, have been shown to reduce prostate volume and prevent the risks of acute urinary retention and need for BPH surgery.

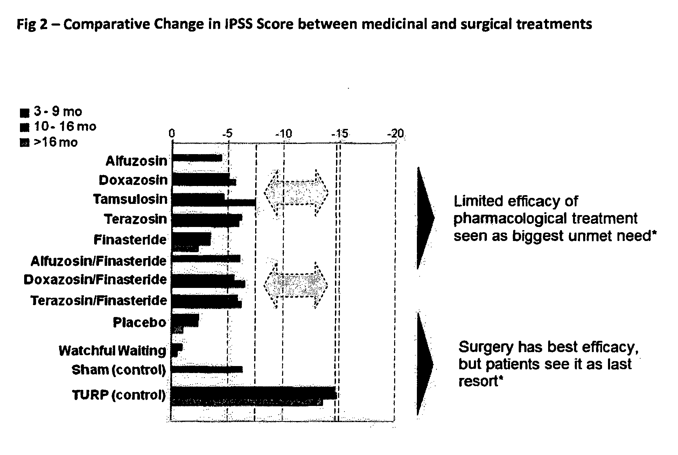

Although the currently available drugs may significantly improve lower urinary tract symptoms in BPH, there is a substantial number of patients who do not benefit from or tolerate the treatment. The magnitude of improvement with current medicinal therapy (as measured by IPSS score, see below) does not compare to the results achieved with surgical treatments (see Fig 2). There is therefore a need for an improved medicinal therapy, which is well tolerated and comparable or more comparable with surgery in terms of efficacy. Furthermore, the currently available drugs are associated with known side effects that sometime lead to treatment discontinuation or lack of compliance. These include notably dizziness and retrograde ejaculation for α-blockers and impotence and loss of libido for 5-α-reductase inhibitors. Accordingly, the need for medicinal therapy with an improved safety profile and/or improved patient compliance also exists.

### Summary of the Invention

According to the present invention in a first aspect there is provided a pharmaceutical composition for use in the treatment of benign prostate hyperplasia; the pharmaceutical composition comprising 9 mg to 40 mg degarelix or pharmaceutically acceptable salt thereof (e.g. acetate); and a solvent; wherein the concentration of the degarelix or salt thereof in the solvent is from 35 to 45 mg/mL (for example, 40 mg/mL).

The composition may be for administration by injection (e.g. by syringe, e.g. by double chamber syringe, or double chamber cartridge e.g. for an injector "pen", as are well known in the art).

Preferably the composition is for administration as a single dose. The composition may be administered in one or more doses, for example two doses, separated, for example, by an interval of 1 to 21 days, for example 14 days.

The composition may further comprise an excipient (e.g. a sugar, e.g. a sugar alcohol, e.g. mannitol). The degarelix or salt thereof may be a colyophilisate with the excipient. The solvent may be, for example, water, or a mixture of water and mannitol. The composition may comprise, for example, 10 mg, 15 mg, 16 mg, 20 mg, 24 mg, 25 mg, 30 mg, 32 mg, 36 mg or 40 mg, of degarelix or salt thereof. The composition may comprise, for example, from 9 to 33 mg degarelix or salt thereof, for example 10 to 30 mg degarelix or salt thereof, for example 12 to 28 mg degarelix or salt thereof, for example 15 to 25 mg degarelix or salt thereof, for example 17 to 23 mg degarelix or salt thereof. The composition may comprise, for example, from 10 to 40 mg degarelix or salt thereof. The composition may be at a concentration of degarelix (or salt thereof) in the solvent of 35, 36, 37, 38, 39, 40, 41, 42, 43, 44 or 45mg/mL, preferably 40 mg/mL. Preferred compositions according to the invention include 10 mg, 16 mg, 20 mg, 30 mg or 32 mg degarelix (e.g. as degarelix acetate); and solvent (e.g. water), wherein concentration of the degarelix (e.g. acetate) in the solvent is 40 mg/mL.

The present applicants have developed degarelix, a synthetic decapeptide antagonist of GnRH which has been approved for use in treatment of prostate cancer. An application for marketing authorisation/new drug application for a formulation for monthly administration was submitted to the FDA and EMEA in February 2008. Marketing Authorisation was granted by the FDA on 24 December 2008, and by EMEA on 17 February 2009. The applicants have found that in the treatment of prostate cancer an effective dose is one which reduces the level of testosterone in the serum to below castration levels, that is to below a serum concentration of 50 ng/dL (0.5 ng/L), and maintains plasma testosterone at this level. Such doses are generally in the region of 240 mg or even higher.

The applicants have surprisingly found that a rather lower dose of degarelix (for example in the region 10 mg to 35 mg degarelix) administered as a solution at a concentration in the region of, for example, 40 mg/mL, may be safe and effective in the treatment of benign prostatic hyperplasia. These dose levels may provide therapeutically effective testosterone suppression, while minimising the time that the plasma testosterone is below castrate levels (contrary to the requirements for treatment of prostate cancer), and the associated side effects.

The applicants have found that compositions according to the invention may be effective in the treatment of BPH when administered periodically, for example once every 3 to 18 months, for example once every 6 or 12 months. Thus, in an example, a composition comprising 30 or 32 mg degarelix and water, at a concentration of degarelix in water of 40 mg/mL, is administered once every 6 or 12 months. It will be appreciated that the composition may be administered as, for example, two compositions/doses of 15 mg (or 16 mg) degarelix separated by an interval of, for example, 14 days (with a further administration of composition in either one or two doses after e.g. 12 months).

The term "treatment of benign prostate hyperplasia" herein includes treatment to alleviate one or more lower urinary tract symptoms associated with BPH (e.g. so called "storage symptoms" such as frequency of urination, urgency of urination, dysuria, nocturia, urgency incontinence ; and/or "voiding symptoms" such as poor stream, hesitancy, terminal dribbling, incomplete voiding, overflow incontinence) as indicated by a reduction in the IPSS score as discussed below; treatment to delay or prevent disease progression and/or reduce the risk of acute urinary retention and/or reduce or delay the need for surgery, and/or treatment to reduce volume of the prostate gland, and/or treatment to increase quality of life of the patient (e.g. as indicated by an improvement in the IPSS QOL survey, see below, or an improvement in the BPH Impact Index), and/or treatment to improve (increase) Qmax (see below).

According to the present invention in a further aspect there is provided a (pharmaceutical) preparation comprising 9 mg to 40 mg degarelix or pharmaceutically acceptable salt thereof (e.g. acetate); and a solvent, wherein the concentration of the degarelix or salt thereof in the solvent is from 35 to 45 mg/mL (for example, 40mg/mL). The preparation may further comprise an excipient (e.g. a sugar, e.g. a sugar alcohol, e.g. mannitol). The degarelix or salt thereof my be a colyophilisate with the excipient. The solvent may be, for example, water, or a mixture of water and mannitol. The pharmaceutical preparation may comprise, for example, 10 mg, 15 mg, 16 mg, 20 mg, 24 mg, 25 mg, 30 mg, 32 mg, 36 mg or 40 mg of degarelix or salt thereof. The preparation may comprise, for example, from 9 to 33 mg degarelix or salt thereof, for example 10 to 30 mg degarelix or salt thereof, for example 12 to 28 mg degarelix or salt thereof, for example 15 to 25 mg degarelix or salt thereof, for example 17 to 23 mg degarelix or salt thereof. The preparation may be at a concentration of degarelix (or salt thereof) in the solvent of 35, 36, 37, 38, 39, 40, 41, 42, 43, 44 or 45 mg/mL, preferably 40 mg/mL. Preferred pharmaceutical preparations according to the invention include 10 mg, 16 mg, 20 mg, 30 mg or 32 mg degarelix (e.g. as degarelix acetate); and solvent (e.g. water), wherein concentration of the degarelix (e.g. acetate) in the solvent is 40 mg/mL.

The pharmaceutical preparation may be administered (for the treatment of BPH) periodically, for example once every 3 to 18 months, for example once every six months or once every year.

Preferably the composition is for administration as a single dose. The composition may be administered in one or more doses, for example two doses, separated by an interval of 1 to 21 days, for example 14 days.

According to the present invention in a further aspect there is provided a method of preparing a composition for treating benign prostate hyperplasia comprising: combining at least one first container comprising a composition or pharmaceutical preparation comprising 9 to 40mg degarelix or pharmaceutically acceptable salt thereof; and at least one second container comprising a solvent (e.g. water); wherein the concentration of the degarelix or salt thereof in the solvent is from 35 to 45 mg/mL (for example, 40 mg/mL). The pharmaceutical composition may further comprise an excipient (e.g. a sugar, e.g. a sugar alcohol, e.g. mannitol). The degarelix or salt thereof may be a colyophilisate with the excipient.

### Detailed description of the invention

### Brief Description of the Figures

Figure 1 a is a graphical representation of the IPSS Sum of Scores by visit and dose (initial 42 day study);
Figure 1b is a graphical representation of the change from baseline in IPSS, for the initial 42 day study and including follow up at 6, 9 and 12 months;
Figure 1c states the seven questions (Q1 - Q7) of the IPSS which evaluate symptoms of urinary obstruction (incomplete emptying, frequency, hesitancy, urgency, weak stream, straining, nocturia) over the preceding week, together with, for each question, a graphical representation of the IPSS score, by visit and dose (initial 42 day study);
Figure 2 is a graphical representation of the comparative change in IPSS Score between medicinal and surgical treatments [source: AUA Guideline on the Management of Benign Prostatic Hyperplasia (2006]; and
Figure 3 is an overview of the pharmacodynamic endpoints derived from the testosterone concentration/time profile.

### Definitions and terms

The term "plasma concentration" herein is used interchangeably with "plasma trough concentration".

The terms "administer", "administration" or "administering" as used herein refer to (i) providing, giving, dosing and/or prescribing degarelix by either a healthcare professional or his or her authorised agent or under his or her direction, and (ii) putting into, taking, or injecting by the patient or person himself or herself, degarelix.

### Degarelix and Related Pharmaceutical Formulations

Degarelix is a potent GnRH antagonist that is an analog of the GnRH decapeptide (pGlu-His-Trp-Ser-Tyr-Gly-Leu-Arg-Pro-Gly -NH₂) incorporating p-ureido-phenylalanines at positions 5 and 6 (Jiang et al. (2001) J. Med. Chem. 44:453-67). Degarelix is a selective GnRH receptor antagonist (blocker) that competitively and reversibly binds to the pituitary GnRH receptors, thereby rapidly reducing the release of gonadotrophins and consequently testosterone (T). It is indicated for treatment of patients with prostate cancer in whom androgen deprivation is warranted (including patients with rising PSA levels after having already undergone prostatectomy or radiotherapy). Unlike GnRH agonists, GnRH receptor blockers do not induce a luteinizing hormone (LH) surge with subsequent testosterone surge/tumor stimulation and potential symptomatic flare after the initiation of treatment.

The active ingredient degarelix is a synthetic linear decapeptide amide containing seven unnatural amino acids, five of which are D-amino acids. The drug substance is an acetate salt, but the active moiety of the substance is degarelix as the free base. The acetate salt of degarelix is a white to off-white amorphous powder (of low density as obtained after lyophilisation). The chemical name is D-Alaninamide, N-acetyl-3-(2-naphthalenyl)-D-alanyl-4-chloro-D-phenylalanyl-3-(3-pyridinyl)-D-alanyl-L-seryl-4-[[[(4S)-hexahydro-2,6-dioxo-4-pyrimidinyl]carbonyl]amino]-L-phenylalanyl-4-[(aminocarbonyl) amino]-D-phenylalanyl-L leucyl-N6-(1-methylethyl)-L-lysyl-L-prolyl. It has an empirical formula of C₈₂H₁₀₃N₁₈O₁₆Cl and a molecular weight of 1,632.3 Da. The chemical structure of degarelix has been previously shown (EP 1003774, US 5,925,730, U.S. 6,214,798) and may be represented by the formula: Ac-D-2Nal-D-4Cpa-D-3Pal-Ser-4Aph(Hor)-D-4Aph(Cbm)-Leu-Lys(iPr)-Pro-D-Ala-NH₂.

### Administration and Dosage

Degarelix may be formulated for administration subcutaneously (as opposed to intravenously), generally in the abdominal region, as described in further detail below. Of course, it may be administered by other methods known in the art (e.g. intramuscular, oral, transdermal etc.). As with other drugs administered by subcutaneous injection, the injection site may vary periodically to adapt the treatment to injection site discomfort. In general, injections should be given in areas where the patient will not be exposed to pressure, e.g. not close to waistband or belt and not close to the ribs.

Administration of degarelix by subcutaneous or intramuscular injection works well, but daily injections are generally not preferred by the patient and so a depot formulation of degarelix may be utilized as described in further detail in WO 03/006049 and U.S. Pub. Nos. 20050245455 and 20040038903. Briefly, subcutaneous administration of degarelix may be conducted using a depot technology in which the peptide is released from a gel-like depot over a period of (typically) one to three months. Degarelix (and related GnRH antagonist peptides) have a high affinity for the GnRH receptor and are much more soluble in water than other GnRH analogues. Degarelix and these related GnRH antagonists are capable of forming a gel after subcutaneous injection, and this gel can act as a depot from which the peptide is released over a period of weeks or even months.

Thus, degarelix may be provided as a powder for reconstitution (with a solvent) as solution for injection (e.g., subcutaneous injection, e.g., to form a depot as described above). The powder may be provided as a lyophilisate containing degarelix (e.g. as acetate) and mannitol. A suitable solvent is water (e.g., water for injection, or WFI). The solvent may be provided in vessels (e.g. vials, prefilled syringes etc.), e.g. containing 5 mL or 6 mL solvent.

In an example, degarelix may be provided in a vial containing 40 mg degarelix (acetate). After reconstitution with about 1.1 mL WFI, there is an extractable volume of 1 mL solution containing about 40 mg degarelix. Administration (e.g. by injection) of 1 mL of the solution provides a dose of (about) 40 mg degarelix at concentration 40 mg/mL; administration (e.g. by injection) of 0.75 mL provides a dose of (about) 30 mg degarelix at concentration 40 mg/mL; administration (e.g. by injection) of 0.625 mL provides a dose of (about) 25 mg degarelix at concentration 40 mg/mL; administration of 0.5 mL provides a dose of (about) 20 mg degarelix at concentration 40 mg/mL; administration (e.g. by injection) of 0.375 mL provides a dose of (about) 15 mg degarelix at concentration 40 mg/mL; administration (e.g. by injection) of 0.25 mL provides a dose of (about) 10 mg degarelix at concentration 40 mg/mL; administration of 0.8 mL provides a dose of (about) 32 mg degarelix at concentration 40 mg/mL; administration (e.g. by injection) of 0.6 mL provides a dose of (about) 24 mg degarelix at concentration 40 mg/mL; administration (e.g. by injection) of 0.4 mL provides a dose of (about) 16 mg degarelix at concentration 40 mg/mL; administration of 0.2 mL provides a dose of (about) 8 mg degarelix at concentration 40 mg/mL; and administration of 0.1 mL provides a dose of (about) 4 mg degarelix at concentration 40 mg/mL.

In another example, degarelix may be provided in a vial containing 120 mg degarelix (acetate) for reconstitution with 3 mL WFI such that each mL of solution contains about 40 mg degarelix; reconstituting gives a 3 mL solution containing about 120 mg degarelix. Administration (e.g. by injection) of 1 mL of the solution provides a dose of (about) 40 mg degarelix at concentration 40 mg/mL; administration (e.g. by injection) of 0.4 mL provides a dose of (about) 16 mg degarelix at concentration 40 mg/mL; administration (e.g. by injection) of 0.8 mL provides a dose of (about) 32 mg degarelix at concentration 40 mg/mL; and administration of 1.6 mL provides a dose of (about) 64 mg degarelix at concentration 40 mg/mL.

The reconstituted solution ready for injection should be perceived as a visually clear liquid.

The dosing regimen for degarelix may be administered as a single dose of, for example, 32 mg administered as 1 injection of 0.8 mL of about 40 mg/mL degarelix formulation. The dose may be repeated after a period of, for example, six or twelve months, thereby providing an "intermittent suppression" treatment of BPH.

Alternatively, the dose ("effective dose") of, for example, 32 mg of degarelix may be administered as a first dose of, for example, 16 mg, administered as 1 injection of 0.4 mL of about 40 mg/mL degarelix formulation; followed after a period of 14 days, by a second dose of, for example, 16 mg, administered as 1 injection of 0.4 mL of about 40 mg/mL degarelix formulation. The "effective dose" of 32 mg may be repeated (either as a single dose, or first and second 16 mg doses), after a period of, for example, twelve months, thereby providing an "intermittent suppression" treatment of BPH.

### Example 1 - Clinical Trial

### Description of Study Design

The study aimed at exploring the potential of degarelix to induce only a short transient lowering of the serum testosterone concentration to or below the castration level, defined as 0.5 ng/mL. Patients diagnosed with BPH were chosen as study subjects in order to capture any signals of efficacy. The study population was men with BPH with: a prostate volume larger than 30 mL, a maximal uroflow of 12 mL/s (with some exceptions), an IPSS score of at least 13, serum prostatic specific antigen (PSA) below 10 ng/mL, and no evidence of prostate cancer. Altogether, 52 patients with BPH were randomly assigned to four parallel groups of 13 patients each.

The patients were randomly allocated to receive either: one injection of 64 mg degarelix on Day 0 ("64 mg", below); one injection of 32 mg degarelix on Day 0 and one on Day 14 ("32 + 32 mg" below); one injection of 32 mg degarelix on Day 0 ("32 mg"); or one injection of 16 mg degarelix on Day 0 and one on Day 14 ("16 + 16 mg").

For each patient and administration, one ampoule of 5 mL of water for injection and one degarelix vial with a total extractable dose of 120 mg was provided. For reconstitution, a volume of 3 mL of water for injection was added to the degarelix vial to obtain a solution with a concentration of 40 mg/mL. The following volumes were administered: 0.4 mL for the (or each) 16 mg administration; 0.8 mL for the (or each) 32 mg administration; or 1.6 mL for the 64 mg administration. The dose was administered to the patient as an abdominal deep subcutaneous injection.

For each patient, the following parameters were measured or assessed (using the methods discussed below), at time points of screening (e.g. -7 days) day 0 ("baseline"), day 14, day 28 and day 42:
1] Patient reported outcomes - the effects of degarelix on urinary morbidity, sexual function and overall quality of life (QoL) due to urinary condition, as assessed using patient reported questionnaires (IPSS, IIEF, and IPSS-global QoL); and
2] Efficacy Endpoints - the measured Peak Urinary Flow, Post-void Residual Volume, and Prostate Volume.

Pharmacodynamic Endpoints were also measured. Quantitative measurement of serum concentrations of testosterone, DHT and SHBG (to calculate free testosterone) during the treatment period was performed in triplicate using a liquid chromatography method (LC-MS/MS), by methods well known in the art. The results for testosterone are discussed below.

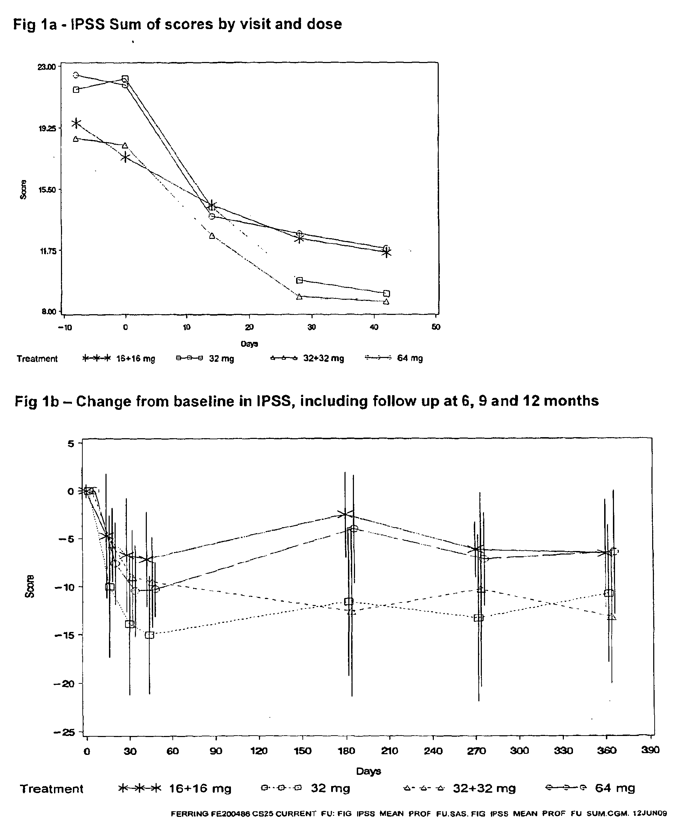

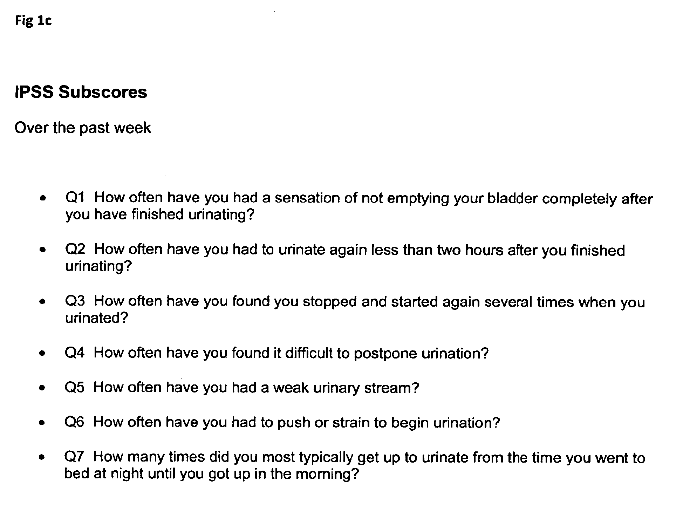

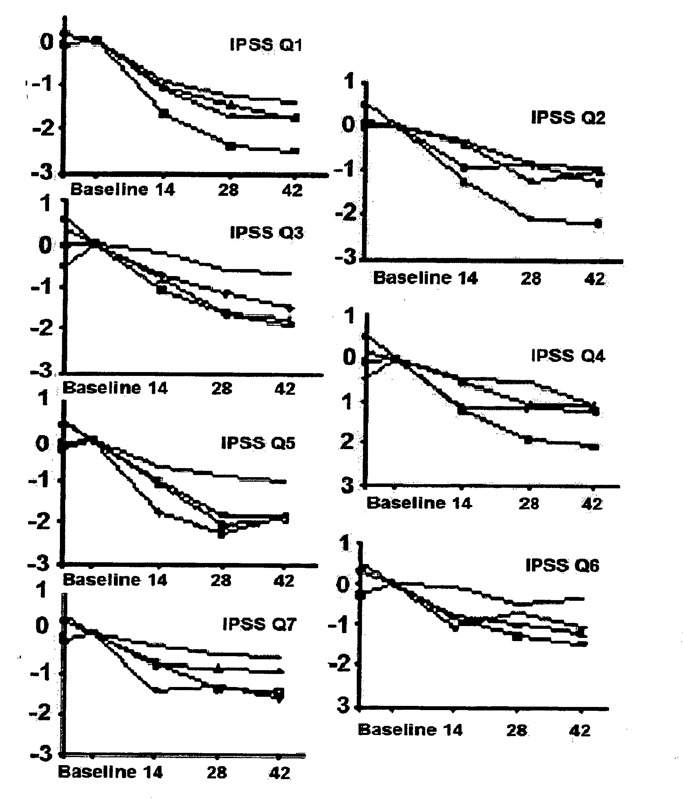

After the initial 6-week treatment period of the exploratory study had been completed, a 12-month follow-up period looking at long-term efficacy commenced. Interim data of the main efficacy variables (IPSS, IPSS Global QoL, prostate volume, urinary flow, and post-void residual volume - measured as set out below) after 6 months' follow-up are also presented herein Table 1, 2, 3 Fig 1 b), together with some IPSS data at 9 and 12 months (Fig 1 b).

### Safety/adverse effects

The adverse events recorded in the study (data not shown) were generally well-known side-effects of the subcutaneous administration of degarelix and did not cause any safety concerns.

### RESULTS

### 1. Patient-reported Outcomes

In order to evaluate the effects of degarelix on urinary morbidity, sexual function and overall quality of life (QoL) due to urinary condition, patient reported questionnaires (IPSS, IIEF, and IPSS-global QoL) were used. The questionnaires were answered by the patient at the clinic visits, in line with standard procedures.

### International Prostate Specific Symptom Score (IPSS) and the global QoL question

The IPSS was developed in 1991 by the American Urological Association to assess the severity of urinary symptoms related to BPH (Barry, M.J., et al., The American Urological Association symptom index for benign prostatic hyperplasia. The Measurement Committee of the American Urological Association. J Urol, 1992. 148(5): p. 1549-57; discussion 1564). It has been widely used in clinical studies, has undergone extensive validation and its psychometric properties are well documented in both the original version (containing a 1-month recall) as well as the 'acute' version used in this study (containing a one-week recall). The WHO has recommended the use of this instrument for the evaluation of BPH and it is considered to be the internationally accepted, standard questionnaire for assessing lower urinary tract symptoms.

The patients were asked to complete the IPSS questionnaire to evaluate their urinary symptoms. The IPSS is a patient-administered questionnaire containing seven items to evaluate symptoms of urinary obstruction (incomplete emptying, frequency, hesitancy, urgency, weak stream, straining, nocturia) over the preceding week. Each urinary symptom question is assigned points from 0 to 5 indicating increasing severity of the particular symptom. The total score can therefore range from 0 to 35 (0-7: mildly symptomatic; 8-19: moderately symptomatic; 20-35: severely symptomatic). A reduction in score over the treatment is indicative of improvement.

Included in the IPSS is one item to evaluate QoL (IPSS-global QoL). The Global QoL Question was developed alongside the IPSS and was officially included as part of the IPSS in 1993 when the WHO formally recommended the IPSS for both symptom and QoL assessment. The Global QoL Question has demonstrated test-retest reliability, internal reliability, construct validity, sensitivity and responsiveness. This question assesses the degree to which patients find their symptoms bothersome. Patients are asked about how they would feel if they were to spend the rest of their lives with their prostate symptoms just as they are now. The choices for answering this quality of life question ranged from "delighted" (rating = 0) to "terrible" (rating = 6).

### The International Index of Erectile Function (IIEF)

The IIEF was originally developed and validated in 1996-1997 with the aim of providing a brief, reliable, self-administered measure of sexual function for use in cross cultural settings detecting treatment-related changes in patients with erectile dysfunction. The instrument was developed via literature review and interviews with patients with erectile dysfunction and their partners, and has been linguistically validated in 37 languages. It has been widely used as a primary endpoint in more than 50 clinical studies-including several BPH studies - and is considered the 'gold standard' measure for efficacy assessment in clinical studies of erectile dysfunction. The IIEF meets psychometric criteria for reliability and validity and has a high degree of sensitivity and specificity.

The IIEF questionnaire evaluated sexual dysfunction associated with treatment. The IIEF is a patient-administered questionnaire containing 15 questions covering five domains: Erectile Function (six questions), Orgasmic Function (two questions), Sexual Desire (two questions), Intercourse Satisfaction (three questions), and Overall Satisfaction (two questions). Question numbers one to ten are scored on a six-point scale from zero ('No sexual activity') to five ('Almost always to always'). Question numbers 11-15 are scored on a five-point scale from one ('Almost never or never') to five ('Almost always to always'). For the Erectile Function domain, a score of one to ten indicates severe erectile dysfunction; 11-16 moderate dysfunction; 17-21 mild to moderate dysfunction; 22-25 mild dysfunction; 26-30 no dysfunction. Also for the other domains a higher score indicates less dysfunction. The IIEF does not yield a total score, only domain scores are calculated.

### Results

### IPSS score

The mean sum of IPSS scores decreased in all treatment groups between all visits subsequent to degerelix administration without any apparent dose or dosing regimen dependency (Table 1, Fig 1a). The mean change from baseline to Day 42 amounted to -6.1 (SD=4.6), -13.2 (SD=6.6), -9.6 (SD=4.7), and -10.0 (SD=5.0) units in the 16+16, 32, 32+32, and 64 mg groups, respectively (Table 1). In other words, the mean IPSS score had improved in all treatment groups on Day 42, albeit somewhat less in the 16+16 mg group compared to the other three groups; improvement was most pronounced in the 32 mg group. An unexpectedly early onset of improvement is indicated as the change from baseline at day 14 (Table 1); the mean IPSS score had improved in all treatment groups on Day 14, albeit rather less in the 16+16 mg group compared to the other three groups; improvement was most pronounced in the 32 mg group.

The 6 month follow up data are discussed below.

Fig 2 shows the Comparative Change in IPSS Score in known medicinal and surgical treatments (AUA Guideline on the Management of Benign Prostatic Hyperplasia (2006). MR, Ad board and Industry reports). This confirms the superiority of surgical intervention, TURP (IPSS change of approximately -14 to -15), over the known medicinal treatments such as Tamsulosin etc (IPSS change of approximately -7.5 maximum, according to the Figure). It can be seen that the mean change of -13.2 shown by the degarelix 32 mg group is comparable with TURP, indicating that degarelix treatment may provide a significant improvement over the currently available treatments such as cetrorelix and ozarelix (Debruyne et al, Cetrorelix pamoate, an LHRH antagonist, in the treatment of BPH: randomized, placebo controlled, multicenter study, Urology, 68 (Supplement 5A). PD-02.11, November 2006; Denes B et al, The efficacy, duration of efficacy and safety of Ozarelix, a novel GnRH antagonist, in men with lower urinary tract symptoms (LUTS) due to benign prostatic hyperplasia (BPH) Urology, 70 (Supplement 3A). MP-20.02, September 2007). The mean change of degarelix -13.2 shown by the degarelix 32 mg group is also significantly better than that shown in a preliminary study with cetrorelix glucoronate, (maximum reduction in mean IPSS between -5.4 to -5.9). (European Urology 54 (2008) 170-180). It should be noted that these are historical comparisons with many limitations; for example, the present study was not placebo controlled. However, the results are indicative of efficacy.

**Table 1 Mean IPSS score and mean change from baseline**

| | **Degarelix 16+16 mg N=13 mean (SD)** | **Degarelix 32 mg N=12 mean (SD)** | **Degarelix 32+32 mg N=12 mean (SD)** | **Degarelix 64 mg N=13 mean (SD)** |
|---|---|---|---|---|
| | | | | |
| Baseline | 17.7 (4.5) | 22.3 (6.5) | 18.2 (5.4) | 21.8 (6.2) |
| | | | | |
| Day 14 | | | | |
| Mean change from baseline | -3.2 (3.4) | - 7.8 (8.7) | -5.5 (5.0) | -8.0(4.0) |
| | | | | |
| | | | | |
| Day 42 | 11.6 (4.5) | 9.1 (6.2) | 8.6 (5.7) | 11.8 (5.9) |
| Mean change | -6.1 (4.6) | -13.2 (6.6) | -9.6 (4.7) | -10.0 (5.0) |
| Percentage responders¹ | 54% | 92% | 83% | 85% |
| | | | | |
| 6 month follow up | | | | |
| Baseline | 18.8 (n=6) | 22.0 (n=7) | 19.3 (n=7) | 20.0 (n=4) |
| Month 6 | 16.3 (4.7) | 10.4 (6.3) | 6.7 (4.5) | 16.0 (8.7) |
| Mean change | -2.5 (4.3) | -11.6 (8.3) | -12.6(9.6) | -4.0(3.6) |
| Percentage responders¹ | 33% | 86% | 86% | 5% |

| | | | | |
|---|---|---|---|---|
| 1. Percentage responders defined as the number of patients that have a decrease in IPSS of at lease 30 % as compared to baseline | | | | |

In line with the decrease in the mean IPSS score, the number of patients in each of the IPSS categories shifted towards mild in all treatment groups, again without any apparent dose or dosing regimen dependency. All patients were categorised as "moderate" (n=27) or "severe" (n=23) at baseline, while on Day 42 only 2 were categorised as "severe", 1 each in the 32 mg and 64 mg groups, 31 as "moderate" and the remaining 17 as "mild".

Figure 1c includes the seven questions (Q1 - Q7) of the IPSS which evaluate symptoms of urinary obstruction (incomplete emptying, frequency, hesitancy, urgency, weak stream, straining, nocturia) over the preceding week. As indicated above, each urinary symptom question is assigned points from 0 to 5 indicating increasing severity of the particular symptom. Figure 1c also includes, for each question, a graphical representation of the IPSS score (as change from baseline), by visit and dose for the initial 42 day study. It can be seen that for Q1 (incomplete emptying), Q2 (frequency of urination), and Q4 (urgency), the reduction in score, as change from baseline, is more marked for the 32 mg dose [the 32 mg score is shown by the bottom line in the graph for each of Q1, Q2 and Q4 at the 28 and 42 day timepoints]. Thus, the 32 mg dose seems to have a pronounced effect on Q1, Q2 and Q4. In other words, the 32 mg dose seems to be associated with a marked improvement on severity of the symptom assessed by each of questions Q1, Q2 and Q4. Q3, Q5, Q6 and Q7 provide further information on urinary characteristics examined.

### IPSS global quality of life (QoL)

The mean IPSS global quality of life (QoL) score decreased in all treatment groups without any apparent dose or dosing regimen dependency (Table 2). The mean (SD) change from baseline to Day 42 was -1.2 (1.4), -2.1 (3.0), -2.4 (1.9), and -2.2 (1.6) in the 16+16, 32, 32+32, and 64 mg groups, respectively.

**Table 2 Mean IPSS global QoL score and mean change from baseline at day 42 and 6 months**

| | **Degarelix 16+16 mg N=13** | **Degarelix 32 mg N=12** | **Degarelix 32+32 mg N=12** | **Degarelix 64 mg N=13** |
|---|---|---|---|---|
| | mean (SD) | mean (SD) | mean (SD) | mean (SD) |
| Baseline | 4.2 (1.1) | 3.8 (1.7) | 4.3 (1.3) | 4.7 (1.4) |
| Day 42 | 2.9 (1.4) | 1.8 (2.0) | 1.9 (1.6) | 2.5 (1.3) |
| Mean change | -1.2 (1.4) | -2.1 (3.0) | -2.4 (1.9) | -2.2 (1.6) |
| | | | | |
| 6-Month Follow-up | | | | |
| Baseline | 4.5 (n=6) | 2.8 (n=7) | 4.3 (n=7) | 4.6 (n=4) |
| Month 6 | 4.2 (1.5) | 1.4 (1.5) | 1.6 (0.8) | 3.8 (1.3) |
| Change from Baseline | -0.3 (0.5) | -1.4 (1.1) | -2.7 (2.1) | -0.8 (1.7) |

The IPSS Global QoL assessment changed in the combined group from 48% feeling unhappy or terrible (scores 5 and 6) and 8% feeling delighted, pleased, or mostly satisfied (scores 0-2) at the baseline assessment, to 58% felling delighted, pleased, or mostly satisfied at the Day 42 assessment with only 12% felling unhappy or terrible.

Interim efficacy data after 6 months' follow-up indicate that the improvements in mean sum of IPSS scores (Table 1, Fig 1b) and mean IPSS Global QoL scores (Table 2) for the 32 mg and 32+32 mg groups were about the same as after 42 days while the improvements in the 16+16 mg and 64 mg groups were much less pronounced. The improvements in mean sum of IPSS scores for the 32 mg and 32+32 mg groups were still indicated at 9 and 12 months (Fig 1b). The sum of IPSS scores findings were also reflected in the percentage of responders; a much higher proportion of the patients in the 32 mg and 32+32 mg groups than in the 16+16 mg and 64 mg groups were responders after 6 months (Table 1). These finding should be carefully interpreted since the number of patients in each group is rather low (n=4-7).

### IIEF score

In general, a mild decrease (i.e. slight worsening) of IIEF scores was observed. These effects are well known side-effects of the androgen deprivation caused by GnRH-receptor antagonists. However, the variation within each of the treatment groups was substantial, and the mean changes in IIEF scale scores were small and much less than the standard deviation. Therefore, interpretation of these results is difficult.

### 2. Efficacy Endpoints: Peak Urinary Flow, Post-void Residual Volume, and Prostate Volume

Peak Urinary Flow, Post-void Residual Volume, and Prostate Volume were assessed in the following order:
Peak urinary flow was determined by flowmetry using the Uropower device (Wiest, World of Medicine AG, Germany). The device fulfilled the International Continence Society standards for maximum urinary flow. The measurement of urinary flow was done in sitting position.

The Post-void Residual Volume (PVR) was evaluated by transabdominal ultrasound. The urine bladder was sonicated from two directions perpendicular to one another resulting in three cursor positions set by the urologist. The volume was calculated automatically. The investigations were performed by urologists by methods known in the art.

The prostate volume was measured by transrectal ultrasound with the patient in lateral position. The prostatic gland was sonicated from two directions perpendicular to one another resulting in three cursor positions set by the urologist. The volume was calculated automatically. The investigations were performed by urologists by methods known in the art.

### Prostate volume compared to baseline

A decrease in mean prostate volume measured by transrectal ultrasound was observed in all treatment groups during the study with the most pronounced percentage changes reported in the 32 mg and 32+32 mg groups, while the smallest change was observed 16+16 mg group (Table 3).

At the 6 months' follow-up visit the differences between the groups in change in mean prostate volume had almost disappeared; only the 64 mg group had a smaller percentage change than the other three groups (Table 3).

**Table 3 Mean Prostate Volume at Baseline, Day 42, and 6 Months and Mean and Percentage Change from Baseline (CS25)**

| | **Degarelix Treatment Group** | | | |
|---|---|---|---|---|
| | **16+16 mg (N=13)** | **32 mg (N=12)** | **32+32 mg (N=12)** | **64 mg (N=13)** |
| | **Mean (SD) Prostate Volume (mL)** | | | |
| Baseline | 42.0 (12.1) | 48.7 (21.0) | 38.1 (7.1) | 38.9 (9.0) |
| Day 42 | 34.5 (11.0) | 34.8 (14.2) | 28.7 (8.2) | 30.6 (10.5) |
| Change from Baseline | -7.5 (7.7) | -13.9 (14.7) | -9.4 (9.3) | -8.2 (10.2) |
| Percentage Change from Baseline 6-Month Follow-up | -17.0 (16.7) | -25.1 (18.0) | -23.5 (20.9) | -19.7 (23.2) |
| Baseline | 40.8 (n=6) | 45.9 (n=7) | 37.1 (n=7) | 38.7 (n=4) |
| Month 6 | 29.6 (10.4) | 34.3 (13.9) | 26.5 (6.0) | 29.0 (3.4) |
| Change from Baseline | -11.2 (4.4) | -11.6 (5.1) | -10.6 (8.8) | -9.7 (10.4) |
| Percentage Change from Baseline | -27.8 (8.7) | -25.6 (8.0) | -27.1 (17.3) | -21.5 (20.6) |

### Maximal Urinary Flow

The Qmax is the maximal urinary flow rate. It is the traditional parameter used in BPH trials and is a good indicator of degree of bladder outlet obstruction (BOO).

The mean maximal urinary flow increased from baseline to Day 42 in the 16+16 mg (from 10.0 to 10.1 mL/s), 32+32 mg (from 9.7 to 12.2 mL/s on Day 42) and in the 64 mg groups (from 9.1 to 11.9 mL/s), while in the 32 mg group the mean urinary flow decreased (from 11.7 to 11.2 mL/s). The intra-group variation in urinary flow was substantial (as reflected in the standard deviations and ranges, which are not shown). In 24 of the patients the maximal urinary flow had increased on Day 42 compared to the baseline, while it decreased in 25 patients and remained unchanged in 1 patient. Twelve patients in the study violated one of the inclusion criteria by having a urinary flow of >12 mL/sec. In addition, the results were apparently variable and very large standard deviations were seen. The mean maximal flow decreased in the two groups given 32 mg degarelix, and increased in the two groups given 64 mg degarelix. However, the changes were small with substantial variation, and moreover, a similar number of patients experienced a decreased flow as experienced an increased flow on Day 42. If the patients that violated the maximal uroflow inclusion criterion are excluded (see above), relatively more patients experienced an increased uroflow, the proportions being 20 to 17. Therefore, it is difficult to draw any conclusions from the changes in mean maximal urinary flow.

### Post-void Residual Volume

The mean post-void residual volume decreased on Day 14 in all treatment groups in an apparent dose dependent way. It further decreased in the 32 mg and in the 32+32 mg group on Day 28, but returned towards, albeit still below, the baseline value on Day 42. The intra-group variation in urinary flow was substantial as reflected in the standard deviations and ranges. In 33 of the patients the post-void residual volume had decreased on Day 42 compared to the baseline, while it increased in 17 patients, distributed in all treatment groups.

Thus, the effects of the treatment on post-void residual volume on Day 42 were less obvious. The mean post-void residual volume demonstrated a time-dependent transient decrease in all groups during the initial 4 weeks, but after 6 weeks it had returned towards the baseline value. Nevertheless, on Day 42 the mean values in all treatment groups were lower than the baseline value, the 32+32 and 64 mg groups showing somewhat smaller post-void residual volume than the 16+16 and 32 mg groups. However, there were no differences between the doses with respect to the proportion of patients that had an increased post-void residual volume.

### 3. Pharmacodynamic Endpoints

Quantitative measurement of serum concentrations of testosterone during the treatment period was performed in triplicate using a liquid chromatography method (LC-MS/MS), by methods well known in the art. The term "baseline", used in relation to the pharmacodynamic endpoint analysis, refers to a measured value for a patient prior to the first administration of degarelix. The term "baseline interval", used in relation to the pharmacodynamic endpoint analysis, refers to an individual baseline value measured prior to the first administration ± 25%, reflecting the intra individual variation in a placebo group observed in a previous degarelix study.

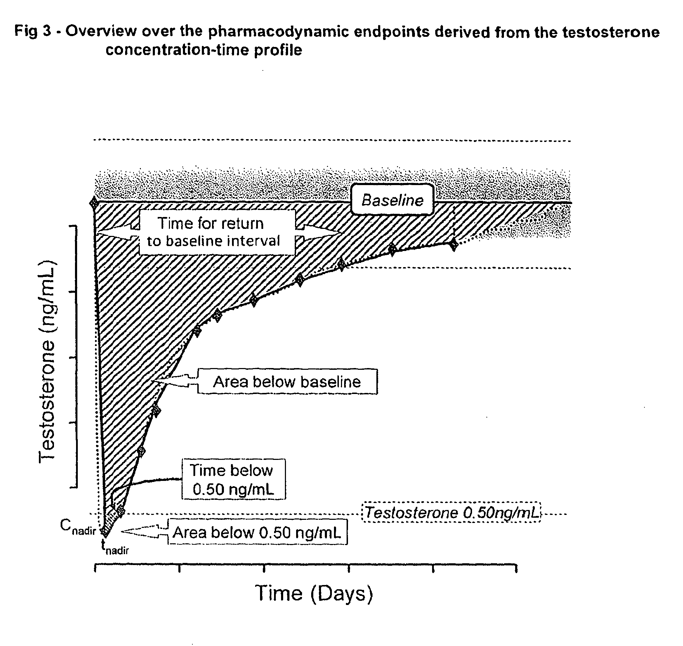

The endpoints related to testosterone concentration-time profiles based on data up to Day 42 that were measured are shown on Fig 3. These include: area of testosterone below the baseline; minimal value of testosterone after each administration of degarelix (Cnadir) and time of Cnadir (tnadir); area of testosterone at testosterone concentration below 0.5 ng/mL; duration of testosterone concentrations below 0.5 ng/mL; time for return to baseline interval. In the Figure 3, the partially shaded area above and below the baseline represents the baseline interval, the upper and lower limits of which are marked by dotted lines.

### Results

The following summarises the results.

### Testosterone Concentration-time Profiles based on Data up to Day 42

A transient testosterone reduction was observed after each of the degarelix administrations, albeit to varying extent and duration between the patients.

### Mean concentrations, Cnadir and tnadir of testosterone after each Degarelix administration

The lowest mean testosterone levels occurred on Day 1 in the 16+16 mg group (1.4 ng/mL, SD 0.5 ng/mL), the 32 mg group (0.9 ng/mL, SD 0.5 ng/mL), and in the 64 mg group (0.4 ng/mL, SD 0.2 ng/mL) while in the 32+32 mg group the lowest mean testosterone level occurred after the second administration, on Day 17 (0.4 ng/mL, SD 0.3 ng/mL).

The greatest change from baseline was -4.0 ng/mL for the 16+16 mg, -4.2 ng/mL for the 32 mg, -4.1 ng/mL for the 32+32 mg, and -4.7 ng/mL for the 64 mg groups, respectively.

Compared to baseline, the mean testosterone level on Day 42 was decreased in the 16+16 mg group (4.5 ng/mL vs. 5.5 ng/mL at baseline), in the 32+32 mg group (2.7 ng/mL vs. 4.5 ng/mL at baseline), and in the 64 mg group (3.0 ng/mL vs. 5.2 ng/mL at baseline) but still above the castration level. In the 32 mg group, the mean testosterone level on Day 42 showed a small increase compared to baseline (5.3 ng/mL vs. 5.2 ng/mL at baseline).

The mean minimal value of testosterone after each administration of degarelix (C_{nadir}) was lower in the higher dose groups than in the lower dose groups: 0.4 ng/mL in the 64 mg group and 0.6 ng/mL (after the first administration) and 0.4 ng/mL (after the second administration) in the 32+32 mg group compared to 0.9 ng/mL in the 32 mg group and 1.4 ng/mL (after the first administration) and 2.0 ng/mL (after the second administration) in the 16+16 mg group.

The corresponding mean time-points (tnadir) to Cnadir were calculated to be earlier in the lower dose groups than in the higher dose groups: 1.1 days after the first administration in the 16+16 mg group and 1.3 days in the 32 mg group compared to 3.2 days after the first administration in the 32+32 mg group and 8.8 days in the 64 mg group (EOT Table 11). After the second administration tnadir increased slightly and was calculated to 4.0 days and 4.3 days for the 16+16 mg and 32+32 mg groups, respectively.

### AUC of testosterone when below the baseline

As expected the largest mean area of testosterone below baseline and baseline interval was observed in the 32+32 mg and 64 mg dose groups. Also the mean time below baseline and baseline interval was longer in these dose groups. The areas in the 16+16 mg and 32 mg, and the 32+32 mg and 64 mg groups, respectively, seemed to be approximately similar, however with large interindividual differences.

### Time with testosterone concentration below 0.5 ng/mL

Individual testosterone concentrations decreased after each administration of degarelix in all subjects in all treatment groups. 22 of the 50 patients showed at one or more time-points testosterone concentrations below the castration level 0.5 ng/mL, the number of patients being 2 (15%), 3 (25%), 9 (75%), and 9 (69%) in the 16+16 mg, 32 mg, 32+32 mg, and 64 mg groups, respectively. In the 16+16 and 32 mg groups the testosterone level recovered above 0.5 ng/mL within 2 days in 4 of the 5 patients, and within 4 days in the remaining patient.

Four of the 9 patients observed with testosterone ≤ 0.5 ng/mL in the 32+32 mg group had concentrations below this level after the first degarelix administration, which in 3 patients recovered to levels above 0.5 ng/mL within 2 days. Five patients were not suppressed below 0.5 ng/mL after the first administration, but reached this level after the second injection. Three subjects had not regained testosterone levels >0.5 ng/mL at Day 42, one of whom had reached testosterone castration levels already after the first degarelix administration.

Five of the 8 patients with testosterone levels ≤ 0.5 ng/mL in the 64 mg group were still below this level on Day 42, while the other three recovered within 5 days.

The mean time of testosterone levels below the castration level 0.5 ng/mL varied substantially between the treatment groups, the 16+16 mg group showing the shortest time and the 64 mg group the longest time.

### Proportion of patients at or below a testosterone level of 0.5 ng/mL at each time-point

At all time-points 32+32 mg and 64 mg dose groups demonstrated a higher proportion of the subjects with testosterone levels below the castration level 0.5 ng/mL compared to the 16+16 mg and 32 mg groups, the highest proportion at all time-points seen after the second administration in the 32+32 mg group, and the sole administration in the 64 mg single dose group. In the 16+16 mg group it was only after the second administration that two patients were suppressed to below 0.5 ng/mL.

There was a slight tendency that the patients experiencing prolonged (i.e. >4 days) castration had a somewhat lower testosterone concentration at baseline, the median being 3.8 ng/mL with a range of 2.2-6.2 ng/mL, compared to the patients with less than 5 days of testosterone concentration below 0.5 ng/mL. However, there was a considerable overlap, exemplified by one patient in the 64 mg group with a baseline value of 3.57 ng/mL not reaching 0.5 ng/mL and another in the same group with a baseline value of 6.23 ng/mL and a prolonged testosterone level below 0.5 ng/mL.

### AUC of testosterone when testosterone level is <0.5 ng/mL

The mean area of testosterone concentrations below 0.5 ng/mL was 0.0 ng*Days/mL in the 16+16 mg group (range 0.0-0.0), 0.0 ng*Days/mL in the 32 mg group (range 0.0-0.1), 2.8 ng*Days/mL in the 32+32 mg group (range 0.0-15.3), and 4.4 ng*Days/mL in the 64 mg group (range 0.0-15.2).

### Time for testosterone to return to baseline interval

The mean time for first return to baseline interval after the first administration was 6.1 (SD=2.8) days in the 16+16 mg group, 9.1 (SD=5.2) days in the 32 mg group, and 9.1 (SD=2.6) days in the 32+32 mg group. The mean time to second return to baseline interval (after the second administration) was 19.5 (SD=33.9) days in the 16+16 mg group and 64.2 (SD=83.5) days in the 32+32 mg group. As several testosterone values did not returned to the baseline interval values until Day 42, the calculated mean time to return to the baseline and baseline interval in the 64 mg group are not applicable. Also after the second administration in the 16+16 mg group and in the 32+32 mg group the calculated times for return to the baseline and baseline intervals are only hypothetical (i.e. calculated, not measured).

### Proportion of patients with testosterone levels at or above the baseline interval at each time point

The overall picture from the analyses of the area below and time to return to the baseline interval was further strengthened by the proportions of patients with a testosterone concentration at or above the baseline interval level at each assessment time. Higher proportions were at earlier timepoints seen in the 16+16 and 32 mg groups compared to the 32+32 and 64 mg groups, and dividing the dose in two injections resulted in more patients returning to the baseline level earlier.

### Discussion

The administration of low doses of degarelix to patients with symptoms of BPH resulted as expected in generally transient decreases in plasma testosterone concentrations. The testosterone decrease was dose related as demonstrated by a more than 2-fold larger area below and longer time to return to the baseline interval in the groups given a total of 64 mg degarelix compared to the total dose 32 mg. However, there were large interindividual differences within the treatment groups.

There was a clear difference between the total doses of degarelix administered with respect to the number of patients that experienced suppressed testosterone concentrations below the castration level 0.5 ng/mL, 64 mg resulting in higher frequency and longer suppression times than 32 mg. In addition there seemed to be a higher frequency and longer suppression times if the dose was given in a single injection compared to if the dose was given in two injections two weeks apart. This is consistent with the degarelix concentration profiles (data not shown), showing higher Cmax and AUC values after the all-in-one injection compared to the dose divided in two injections. From a "no castration"-perspective, the 16+16 mg administration was the most favourable dosing regimen, especially if the results from the first dose of the 32+32 mg are combined with the single dose 32 mg data, the former resulting in 2 (15%) patients castrated for less than 2 days, and the latter resulting in 8 (33%) patients castrated for generally 2-4 days.

### Conclusions

The overall IPSS score and the IPSS global QoL score were substantially improved in all treatment groups, indicating beneficial effects Unexpectedly, an improvement was found early on in treatment (14 days). All treatment groups demonstrated a clear effect on the prostate volume, a small effect on the post-void residual volume, and a doubtful effect on the maximal urinary flow. The 16+16 mg group showed the smallest effect in all efficacy parameters compared to the other three treatment groups.

Administration of degarelix induced decreased levels of testosterone in all patients. The 16+16 and 32 mg degarelix administrations caused transient testosterone concentrations below the castration level 0.5 ng/mL in few patients, while 32+32 and 64 mg administrations caused longer sub-castration concentration periods in more patients. The IIEF scores indicated a slight worsening of the erectile function in all treatment groups, most notably among patients with testosterone levels below the castration level on Day 42.

While all treatment regimes showed beneficial effects, the potential for negative effects associated with the 64 mg and 32 + 32 mg indicate that a preferred range is in the region 9 to 40 mg degarelix (per dose).

There were no safety concerns at any dose or dosing regimen.

## Claims

1. A pharmaceutical composition for use in the treatment of benign prostate hyperplasia; the pharmaceutical composition comprising 9 mg to 40 mg degarelix or pharmaceutically acceptable salt thereof; and a solvent; wherein the concentration of the degarelix or salt thereof in the solvent is from 35 to 45 mg/mL.

2. A pharmaceutical composition for use according to claim 1 comprising 9 to 33 mg degarelix or pharmaceutically acceptable salt thereof.

3. A pharmaceutical composition for use according to claim 1 comprising 10 to 40 mg degarelix or pharmaceutically acceptable salt thereof.

4. A pharmaceutical composition for use according to any preceding claim wherein the solvent is water.

5. A pharmaceutical composition for use according to any preceding claim wherein concentration of degarelix (or salt thereof) in the solvent is 40 mg/mL.

6. A pharmaceutical composition for use according to any preceding claim further comprising an excipient.

7. A pharmaceutical composition for use according to claim 6 wherein the degarelix or salt thereof is a colyophilisate with the excipient.

8. A pharmaceutical composition for use according to claim 6 or 7 wherein the excipient is mannitol.

9. A pharmaceutical preparation comprising 9 mg to 40 mg degarelix or pharmaceutically acceptable salt thereof; and a solvent, wherein the concentration of the degarelix or salt thereof in the solvent is from 35 to 45 mg/mL.

10. A preparation according to claim 9 comprising 9 to 33 mg degarelix or pharmaceutically acceptable salt thereof.

11. A preparation according to claim 9 comprising 10 to 40 mg degarelix or pharmaceutically acceptable salt thereof.

12. A preparation according to any of claims 9 to 11 further comprising an excipient.

13. A preparation according to claim 12 wherein the degarelix or salt thereof is a colyophilisate with the excipient.

14. A preparation according to claim 12 or 13 wherein the excipient is mannitol.

15. A preparation according to any of claims 12 to 14 wherein the solvent is water.

16. A preparation according to any of claims 12 to 15 wherein the concentration of degarelix (or salt thereof) in the solvent is 40 mg/mL.

17. A method of preparing a composition for treating benign prostate hyperplasia comprising: combining at least one first container comprising a composition or pharmaceutical preparation comprising 9 mg to 40 mg degarelix or pharmaceutically acceptable salt thereof; and at least one second container comprising a solvent; wherein the concentration of the degarelix or salt thereof in the solvent is from 35 to 40 mg/mL.

## Patentansprüche

1. Pharmazeutische Zusammensetzung zur Verwendung bei der Behandlung von gutartiger Prostatahyperplasie, wobei die pharmazeutische Zusammensetzung 9 mg bis 40 mg Degarelix oder eines pharmazeutisch akzeptablen Salzes davon und ein Lösungsmittel beinhaltet, wobei die Konzentration des Degarelix oder Salzes davon in dem Lösungsmittel 35 bis 45 mg/ml beträgt.

2. Pharmazeutische Zusammensetzung zur Verwendung nach Anspruch 1, die 9 bis 33 mg Degarelix oder eines pharmazeutisch akzeptablen Salzes davon beinhaltet.

3. Pharmazeutische Zusammensetzung zur Verwendung nach Anspruch 1, die 10 bis 40 mg Degarelix oder eines pharmazeutisch akzeptablen Salzes davon beinhaltet.

4. Pharmazeutische Zusammensetzung zur Verwendung nach einem vorherigen Anspruch, wobei das Lösungsmittel Wasser ist.

5. Pharmazeutische Zusammensetzung zur Verwendung nach einem vorherigen Anspruch, wobei die Konzentration von Degarelix (oder einem Salz davon) in dem Lösungsmittel 40 mg/ml beträgt.

6. Pharmazeutische Zusammensetzung zur Verwendung nach einem vorherigen Anspruch, die ferner einen Exzipienten beinhaltet.

7. Pharmazeutische Zusammensetzung zur Verwendung nach Anspruch 6, wobei das Degarelix oder Salz davon ein Colyophilisat mit dem Exzipienten ist.

8. Pharmazeutische Zusammensetzung zur Verwendung nach Anspruch 6 oder 7, wobei der Exzipient Mannitol ist.

9. Pharmazeutisches Präparat, das 9 mg bis 40 mg Degarelix oder eines pharmazeutisch akzeptablen Salzes davon und ein Lösungsmittel beinhaltet, wobei die Konzentration von Degarelix oder einem Salz davon in dem Lösungsmittel 35 bis 45 mg/ml beträgt.

10. Präparat nach Anspruch 9, das 9 bis 33 mg Degarelix oder eines pharmazeutisch akzeptablen Salzes davon beinhaltet.

11. Präparat nach Anspruch 9, das 10 bis 40 mg Degarelix oder eines pharmazeutisch akzeptablen Salzes davon beinhaltet.

12. Präparat nach einem der Ansprüche 9 bis 11, das ferner einen Exzipienten beinhaltet.

13. Präparat nach Anspruch 12, wobei das Degarelix oder Salz davon ein Colyophilisat mit dem Exzipienten ist.

14. Präparat nach Anspruch 12 oder 13, wobei der Exzipient Mannitol ist.

15. Präparat nach einem der Ansprüche 12 bis 14, wobei das Lösungsmittel Wasser ist.

16. Präparat nach einem der Ansprüche 12 bis 15, wobei die Konzentration von Degarelix (oder einem Salz davon) in dem Lösungsmittel 40 mg/ml beträgt.

17. Verfahren zur Herstellung einer Zusammensetzung zum Behandeln von gutartiger Prostatahyperplasie, das Folgendes beinhaltet: Kombinieren von wenigstens einem ersten Behälter, der eine Zusammensetzung oder ein pharmazeutisches Präparat beinhaltet, die/das 9 mg bis 40 mg Degarelix oder eines pharmazeutisch akzeptablen Salzes davon beinhaltet, und wenigstens einem zweiten Behälter, der ein Lösungsmittel beinhaltet, wobei die Konzentration von Degarelix oder dem Salz davon in dem Lösungsmittel 35 bis 40 mg/ml beträgt.

## Revendications

1. Composition pharmaceutique à utiliser dans le traitement de l'hyperplasie bénigne de la prostate; la composition pharmaceutique comprenant de 9 mg à 40 mg de dégarelix ou d'un sel pharmaceutiquement acceptable de celui-ci; et un solvant; dans laquelle la concentration du dégarelix ou d'un sel de celui-ci dans le solvant est de 35 à 45 mg/ml.

2. Composition pharmaceutique à utiliser selon la revendication 1, comprenant 9 à 33 mg de dégarelix ou d'un sel pharmaceutiquement acceptable de celui-ci.

3. Composition pharmaceutique à utiliser selon la revendication 1, comprenant 10 à 40 mg de dégarelix ou d'un sel pharmaceutiquement acceptable de celui-ci.

4. Composition pharmaceutique à utiliser selon l'une quelconque des revendications précédentes, dans laquelle le solvant est de l'eau.

5. Composition pharmaceutique à utiliser selon l'une quelconque des revendications précédentes, dans laquelle la concentration de dégarelix (ou d'un sel de celui-ci) dans le solvant est de 40 mg/ml.

6. Composition pharmaceutique à utiliser selon l'une quelconque des revendications précédentes, comprenant en outre un excipient.

7. Composition pharmaceutique à utiliser selon la revendication 6, dans laquelle le dégarelix ou le sel de celui-ci est un produit de co-lyophilisation avec l'excipient.

8. Composition pharmaceutique à utiliser selon la revendication 6 ou 7, dans laquelle l'excipient est du mannitol.

9. Préparation pharmaceutique comprenant de 9 mg à 40 mg de dégarelix ou d'un sel pharmaceutiquement acceptable de celui-ci; et un solvant, dans laquelle la concentration du dégarelix ou d'un sel de celui-ci dans le solvant est de 35 à 45 mg/ml.

10. Préparation selon la revendication 9, comprenant 9 à 33 mg de dégarelix ou d'un sel pharmaceutiquement acceptable de celui-ci.

11. Préparation selon la revendication 9, comprenant 10 à 40 mg de dégarelix ou d'un sel pharmaceutiquement acceptable de celui-ci.

12. Préparation selon l'une quelconque des revendications 9 à 11, comprenant en outre un excipient.

13. Préparation selon la revendication 12, dans laquelle le dégarelix ou sel de celui-ci est un produit de co-lyophilisation avec l'excipient.

14. Préparation selon la revendication 12 ou 13, dans laquelle l'excipient est du mannitol.

15. Préparation selon l'une quelconque des revendications 12 à 14, dans laquelle le solvant est de l'eau.

16. Préparation selon l'une quelconque des revendications 12 à 15, dans laquelle la concentration de dégarelix (ou d'un sel de celui-ci) dans le solvant est de 40 mg/ml.

17. Procédé de préparation d'une composition pour traiter l'hyperplasie bénigne de la prostate, comprenant : combiner au moins un premier récipient comprenant une composition ou une préparation pharmaceutique comprenant de 9 mg à 40 mg de dégarelix ou d'un sel pharmaceutiquement acceptable de celui-ci; et au moins un deuxième récipient comprenant un solvant; dans lequel la concentration du dégarelix ou d'un sel de celui-ci dans le solvant est de 35 à 40 mg/ml.
